Europäisches Patentamt

(19) European Patent Office

Office européen des brevets

(11) **EP 0 602 029 B1**

(12) **FASCICULE DE BREVET EUROPEEN**

(45) Date de publication et mention
de la délivrance du brevet:
**06.03.1996 Bulletin 1996/10**

(21) Numéro de dépôt: **91916145.5**

(22) Date de dépôt: **04.09.1991**

(51) Int. Cl.$^6$: **A61K 7/48**, A61K 7/06,
A61K 35/78

(86) Numéro de dépôt international: **PCT/FR91/00706**

(87) Numéro de publication internationale:
**WO 93/04667 (18.03.1993 Gazette 1993/08)**

(54) **COMPOSITION COSMETIQUE OU PHARMACEUTIQUE CONTENANT UN EXTRAIT DE COLEUS ESQUIROLII, DE COLEUS SCUTELLARIOIDES OU DE COLEUS XANTHANTHUS**

KOSMETISCHES ODER PHARMAZEUTISCHES MITTEL, DAS EIN COLEUS ESQUIROLII, COLEUS SCUTELLARIOIDES ODER COLEUS XANTHANTHUSEXTRAKT ENTHÄLT

COSMETIC OR PHARMACEUTICAL COMPOSITION CONTAINING AN EXTRACT OF COLEUS ESQUIROLLII, COLEUS SCUTELLARIOIDES OR COLEUS XANTHANTHUS

(84) Etats contractants désignés:
**BE CH DE ES FR GB IT LI**

(43) Date de publication de la demande:
**22.06.1994 Bulletin 1994/25**

(73) Titulaire: **LVMH RECHERCHE
F-92752 Nanterre (FR)**

(72) Inventeurs:
• **MEYBECK, Alain
F-92400 Courbevoie (FR)**
• **BONTE, Frédéric
F-92400 Courbevoie (FR)**
• **DUMAS, Marc
F-92700 Colombes (FR)**

(74) Mandataire: **Portal, Gérard
Cabinet Beau de Loménie
158, rue de l'Université
F-75340 Paris Cédex 07 (FR)**

(56) Documents cités:
**EP-A- 0 293 837          WO-A-91/02516
GB-A- 1 589 326**

• **STN Serveur de Bases de Données, Karlsruhe, DE, Fichier Biosis, numero d'access AN=75-19643; O.K. HOLDSWORTH: "Some medicinal plants of the Marawaka Kukukuku people", & Sci. New Guinea 1(3-4), 1973, voir le résumé**

**Description**

La présente invention concerne essentiellement une composition cosmétique ou pharmaceutique, notamment dermatologique, destinée en particulier à favoriser la pigmentation de la peau ou des cheveux, contenant un extrait de Coleus Esquirolii, de Coleus Scutellarioides, de Coleus Xanthanthus, et son procédé de préparation.

Elle concerne également l'utilisation d'un extrait de Coleus Esquirolii, de Coleus Scutellarioides, de Coleus Xanthanthus pour la préparation d'une telle composition cosmétique ou pharmaceutique.

Les espèces Coleus font partie de la famille des Labiacées, tout comme les Plectranthus qui en sont très proches, et sont souvent confondues entre elles, étant en outre généralement originaires des mêmes régions. Ainsi, l'espèce Coleus barbatus est généralement désignée sous le nom de Plectranthus barbatus, et l'espèce Coleus aromaticus a pour autre nom Plectranthus aromaticus. Il existe près de 200 espèces de Coleus réparties dans les régions tropicales et substropicales d'Asie, d'Afrique, d'Australie et des îles pacifiques. Environ 9 espèces sont répertoriées en Inde. Les 10 variétés principales de Coleus sont répertoriées dans les dictionnaires indiens et en particulier "The Wealth of India, a dictionary of Indian Raw materials and Industrial Products, Raw materials", volume II, Dehli 1950, pages 308-309 ; le livre intitulé "Indian Materia Medica" du Docteur K.M. Nadkarni's, troisième édition révisée et complétée par A.K. Nadkarni en deux volumes, volume I, page 372 ; le livre intitulé "the Flora of British India" par Sir J.D. Hooker, C.B., K.C., S.I. volume IV ayant pour titre "Asclepiadae to Amarantaceae", pages 624 à 627.

L'utilisation de Coleus forskholii comme matière première pour une extraction de la forskoline est déjà connue notamment à partir du document FR-A-2 336 138. La forskoline est également connue comme ayant une activité amincissante par une publication de GREENWAY dans EP-190 165.

Les documents FR-A-2 336 138 et EP-A1-0 243 646 décrivent également un certain nombre de propriétés pharmacologiques de ces substances, telles qu'une activité hypotensive et calmante du système nerveux central.

Le document WO-A-91/02516 décrit des extraits de Coleus contenant la forskoline, ayant une action de stimulation de la mélanogénèse et de pigmentation attribuée aux forskolines.

La présente invention est basée sur la découverte que des extraits de Coleus Esquirolii, de Coleus Scutellarioides, ou de Coleus Xanthanthus, bien que ne contenant pas de forskoline, présentent des propriétés biologiques intéressantes utilisables dans les domaines cosmétique et pharmaceutique. Il a notamment été observé par les inventeurs que ces extraits possédaient de façon inattendue une activité stimulante de la mélanogénèse au niveau des mélanocytes présents dans la peau ou les follicules pileux, et permettaient ainsi de favoriser la pigmentation de la peau ou des cheveux, ainsi que de réaliser un traitement des désordres de la pigmentation de la peau et des cheveux en favorisant plus particulièrement la biosynthèse de mélanine.

La présente invention a donc pour but de résoudre le nouveau problème technique consistant en la fourniture d'une nouvelle composition cosmétique ou pharmaceutique, notamment dermatologique, destinée en particulier à favoriser la pigmentation de la peau ou des cheveux.

La présente invention a encore pour but de résoudre le nouveau problème technique consistant en la préparation d'une nouvelle formulation d'une composition cosmétique ou pharmaceutique présentant une bonne activité de stimulation de la mélanogénèse au niveau des mélanocytes présents dans la peau ou dans les follicules pileux.

La présente invention a encore pour but de fournir une solution au nouveau problème technique consistant en la fourniture d'un extrait de plante particulièrement aisé à obtenir, qui présente en lui-même une bonne activité stimulante de la mélanogénèse au niveau des mélanocytes présents dans la peau ou Les follicules pileux, sans avoir à réaliser l'isolation d'une substance active quelconque, ces procédés d'isolation étant en général longs et coûteux.

Tous ces nouveaux problèmes techniques sont résolus pour la première fois par la présente invention d'une manière satisfaisante, utilisable à l'échelle industrielle.

Ainsi, selon un premier aspect, la présente invention concerne une composition cosmétique ou pharmaceutique, notamment dermatologique, caractérisée en ce qu'elle comprend à titre d'ingrédient actif un extrait de Coleus Esquirolii, de Coleus Scutellarioides, ou de Coleus Xanthanthus, ou l'un de leurs mélanges.

Selon un second aspect étroitement lié au premier aspect, la présente invention concerne une composition cosmétique ou pharmaceutique, notamment dermatologique, destinée à favoriser la pigmentation de la peau ou des cheveux, caractérisée en ce qu'elle comprend à titre d'ingrédient actif un extrait de Coleus Esquirolii, de Coleus Scutellarioides, ou de Coleus Xanthanthus, ou l'un de leurs mélanges.

Selon une autre caractéristique, on utilise un extrait organique de Coleus Esquirolii, de Coleus Scutellarioides, ou de Coleus Xanthanthus que l'on trouve notamment en Inde. Cet extrait est obtenu avantageusement par un procédé comprenant au moins une étape d'extraction avec un solvant choisi parmi le groupe constitué par l'acétate d'éthyle, le méthanol, l'éthanol et le dichlorométhane. D'une façon générale on peut utiliser comme solvant, des solvants organiques, tels que les hydrocarbures aromatiques, des hydrocarbures aliphatiques ou aromatiques halogénés, des éthers dialcoyliques, des dialcoyl-cétones, des alcanols, des acides carboxyliques et leurs esters ou d'autres solvants, comme le diméthylformamide, le dioxanne, le tétrahydrofuranne et le diméthylsulfoxyde. Parmi les solvants précités, des solvants préférés sont le benzène, le toluène ou le xylène, le chlorure de méthylène, le chloroforme, l'acétate d'éthyle, le méthanol ou l'éthanol. Le rapport de la matière végétale à l'agent d'extraction n'est pas critique et généralement sera compris entre 1 : 5 et 1 : 20 parties en poids, et il est préférentiellement de 1 : 10 environ. L'extraction s'effectue à des températures comprises entre la température ambiante et le point d'ébullition du solvant utilisé pour l'extraction. Une technique avantageuse d'extraction est la technique dite d'extraction au Soxhlet. Il peut être avantageux et dans certains cas nécessaire d'évaporer le solvant par exemple par lyophilisation et de reprendre les extraits bruts en vue d'une purification. Dans le cadre de la présente invention, l'extraction alcoolique est particulièrement intéressante, notamment en fin de procédure d'obtention de l'extrait en raison du caractère habituellement peu toxique des alcools. Ainsi un alcool particulièrement avantageux est l'éthanol.

Un autre solvant particulièrement avantageux est l'acétate d'éthyle, parce qu'il fournit un extrait de bonne efficacité.

D'une façon générale, la concentration des extraits utilisés conformément à la présente invention, pour la préparation d'une composition cosmétique ou pharmaceutique, exprimée en poids sec est comprise entre 0,001% et 2% en poids, de préférence entre 0,01% et 0,5% en poids, par rapport au poids total de la composition.

Les compositions cosmétiques ou pharmaceutiques, notamment dermatologiques, selon la présente invention, peuvent être appliquées par voie topique pour favoriser la pigmentation de la peau et des cheveux, en particulier dans des compositions se. présentant sous forme de crèmes, de gels ou de lotions destinées à l'application sur la peau ou les cheveux.

Ainsi, les compositions cosmétiques ou pharmaceutiques, notamment dermatologiques, selon l'invention trouvent diverses applications en cosmétologie ou en dermatologie, en particulier lorsque l'augmentation de la pigmentation est recherchée. Par exemple, ces compositions peuvent être utilisées comme produits solaires pour accélérer ou intensifier le bronzage, ce qui, outre l'avantage esthétique souvent recherché, permet de renforcer les défenses naturelles contre le rayonnement ultraviolet par l'augmentation du taux de mélanine dans l'épiderme. Ces compositions peuvent encore être utilisées, par exemple sous forme de crèmes, pour donner à la peau un aspect plus hâlé, ou encore, sous forme de lotions, pour la prévention et le traitement de l'apparition des cheveux gris. Par ailleurs, en dermatologie, les compositions selon la présente invention peuvent être utilisées comme agents thérapeutiques, seules ou en association avec d'autres médicaments, en particulier en administration topique dans le traitement des disfonctionnements de la mélanogénèse.

Selon un mode de réalisation avantageux, une composition cosmétique ou pharmaceutique selon l'invention contient en outre une xanthine, en particulier l'IBMX (isobutylméthylxanthine) ou la théophylline, de préférence à une concentration pondérale comprise entre 0,01% et 2%, et encore de préférence comprise entre 0,01% et 0,5%, par rapport au poids total de la composition.

Selon un autre mode de réalisation, une composition cosmétique ou pharmaceutique salon l'invention contient en outre de la tyrosine ou l'un de ses dérivés, de préférence à une concentration pondérale comprise entre 0,001% et 10%, par rapport au poids total de la composition.

Selon encore un autre mode de réalisation, une composition cosmétique ou pharmaceutique selon l'invention contient en outre au moins une autre substance active, à une concentration efficace, choisie parmi les vitamines, en particulier les vitamines B, la quinine ou ses dérivés, les rubéfiants, tels que le nicotinate de méthyle, un surnageant de culture de fibroblastes de papilles, tel que défini dans le document EP-A-272 920, des hydrolysats de kératine, des oligo-éléments tels que zinc, sélénium, cuivre, des inhibiteurs de 5 $\alpha$-réductase, tels que progestérone, cyprotérone acétate, minoxidil, acide azélaïque et ses dérivés, un 4-méthyl-4-azastéroïde, en particulier la 17-$\beta$-N,N-diéthylcarbamoyl-4-méthyl-4-aza-5-$\alpha$-androstan-3-one, ou encore un extrait de Serenoa repens.

Selon un troisième aspect, la présente invention concerne encore un procédé de fabrication d'une composition cosmétique ou pharmaceutique, notamment dermatologique, destinée en particulier à favoriser la pigmentation de la peau ou des cheveux, caractérisé en ce qu'il comprend l'incorporation d'au moins un extrait de Coleus Esquirolii, de Coleus Scutellarioides, ou de Coleus Xanthanthus ou d'un de leurs mélanges, tel que précédemment décrit dans un excipient, véhicule ou support cosmétiquement ou pharmaceutiquement acceptable.

Selon un quatrième aspect, l'invention concerne l'utilisation d'un extrait de Coleus Esquirolii, de Coleus Scutellarioides, ou Coleus Xanthanthus, ou l'un de leurs mélanges pour la préparation d'une composition cosmétique ou pharmaceutique, notamment dermatologique, destinée en particulier à favoriser la pigmentation de la peau ou des cheveux et/ou au traitement des désordres de la pigmentation de la peau et des cheveux.

Enfin, selon un dernier aspect, la présente invention concerne encore un procédé de traitement cosmétique de la peau ou ces cheveux pour favoriser la pigmentation, caractérisé en ce qu'il comprend l'application en une quantité efficace pour réaliser une pigmentation, d'au moins un extrait de Coleus Esquirolii, de Coleus Scutellarioides, ou Coleus Xanthanthus, ou l'un de leurs mélanges, tel que précédemment décrit, incorporé dans un excipient, véhicule ou support cosmétiquement acceptable.

Dans tous les aspects précédents de la présente invention, on peut incorporer l'extrait de Coleus précité dans des phases lamellaires lipidiques hydratées ou dans des liposomes.

Le terme "lipidique" dans l'expression "phase lamellaire lipidique" couvre toutes les substances comprenant une chaîne carbonée dite grasse, comportant généralement plus de 5 atomes de carbone, cette substance étant habituellement dénommée "lipide".

Selon l'invention, on utilise à titre de lipide, pour former les phases lamellaires lipidiques, ou les liposomes, des lipides amphiphiles, c'est-à-dire constitués de moléculaires possédant un groupe hydrophile indifféremment ionique ou non ionique et un groupe lipophile, ces lipides amphiphiles étant susceptibles de former des phases lamellaires lipidiques ou des liposomes, en présence d'une phase aqueuse, selon la quantité d'eau dans le mélange.

En particulier, parmi ces lipides, on peut citer : les phospholipides, les phosphoaminolipides, les glycolipides, les alcools gras polyoxyéthylénés, les esters de polyol éventuellement polyoxyéthylénés. De telles substances sont par exemple constituées par une lécithine d'oeuf ou de soja, une phosphatidyle sérine, une sphyngomyéline, un cérébroside ou un stéarate de polyglycéroloxyéthyléné.

De préférence, selon l'invention, on utilise un mélange lipidique constitué d'au moins un lipide amphiphile et d'au moins un lipide hydrophobe tel que stérol, comme le cholestérol ou le $\beta$-sitostérol. La quantité, exprimée en mole, de lipide hydrophobe ne doit généralement pas être supérieure à la quantité de lipide amphiphile, et de préférence elle ne doit pas être supérieure à 0,5 fois cette quantité.

L'incorporation en des phases lamellaires lipidiques hydratées ou dans des liposomes, des composés ou des extraits contenant ces composés, utilisés conformément à la présente invention, peut être réalisée selon des techniques de préparation connues, décrites par exemple dans le document EP-B1-0 087 993, éventuellement en combinaison avec le document EP-B1-0 107 559.

D'autres buts, caractéristiques et avantages de l'invention apparaîtront clairement à la lumière de la description explicative qui va suivre faite en référence à plusieurs exemples donnés simplement à titre d'illustration et qui ne saurait donc en aucune façon limiter la portée de l'invention.

Dans les exemples, les pourcentages sont donnés en poids, sauf indication contraire.

## EXEMPLE 1

### Obtention d'un extrait de Coleus à partir de Coleus Esquirolii

On traite 500 g de plantes entières de Coleus Esquirolii selon la technique d'extraction au Soxhlet avec une quantité suffisante de méthanol pour baigner complètement la plante, au reflux pendant 3 h.

On filtre les extraits méthanoliques et on évapore le filtrat méthanolique sous pression réduite en obtenant ainsi l'extrait de Coleus Esquirolii appelé extrait $I_1$.

## EXEMPLE 2

En opérant comme décrit à l'exemple 1, mais en utilisant comme plante le Coleus Scutellarioides et comme solvant l'acétate d'éthyle, on obtient un extrait de Coleus Scutellarioides dénommé extrait $I_2$.

## EXEMPLE 3

### Obtention d'un extrait de Coleus Xanthanthus

On procède comme décrit à l'exemple 1 si ce n'est qu'on utilise comme plante le Coleus Xanthanthus, le solvant restant le méthanol.

On obtient ainsi un extrait de Coleus Xanthanthus dénommé extrait $I_3$.

## EXEMPLE 4

Des racines séchées et finement broyées de Coleus Esquirolii (1 kg) sont extraites à plusieurs reprises avec du benzène (3 x 5 l) de préférence selon la technique d'extraction au Soxhlet à 70°C pendant plusieurs heures. Les extraits de benzène obtenus sont filtrés et concentrés sous vide pour éliminer le benzène.

## EXEMPLE 5

### Incorporation d'un extrait de Coleus Esquirolii dans des phases lamellaires lipidiques hydratées ou dans des liposomes

Un extrait de Coleus Esquirolii obtenu conformément à l'exemple 1 est incorporé dans des phases lamellaires lipidiques hydratées ou dans des liposomes selon la technique de préparation suivante :
on pèse :

- lécithine de soja : 0,9 g
- $\beta$-sitostérol : 0,1 g

- extrait de Coleus lyophilisé l1 de l'exemple 1 : 0,025 g

Ces constituants sont dissous dans un mélange constitué de 100 ml de dichlorométhane et 25 ml de méthanol.

On évapore le mélange ainsi obtenu dans un ballon rotatif à température de 45°C sous pression réduite.

Le film lipidique ainsi obtenu est alors repris et dispersé sous agitation dans de l'eau distillée qsp 50 g, à température ambiante pendant 2 h.

La suspension de vésicules lipidiques obtenue est ensuite homogénéisée par un traitement aux ultrasons pendant 30 min à 4°C, sous puissance de 150 W.

La taille moyenne des liposomes ainsi obtenus est d'environ 212 nm.

Selon une variante de réalisation avantageuse, on gélifie ensuite cette suspension en la mélangeant à 50 g de gel de carbopol® 904 à 1,25 % préparé séparément de façon classique. On obtient ainsi 100 g environ d'une suspension gélifiée de liposomes encapsulant l'extrait de Coleus Esquirolii, dont la concentration est d'environ 0,025 % par rapport au poids total de la suspension.

## EXEMPLE 6

## Mesure de l'activité d'un extrait de Coleus Esquirolii, de Coleus Scutellarioides ou de Coleus Xanthanthus selon l'invention sur les mélanocytes en culture

## Protocole :

Des mélanocytes murins sont cultivés sur un milieu approprié de manière classique.

Au jour J = 0, le produit à tester est introduit dans le milieu de culture.

Au jour J = 5, on prélève les cellules que l'on isole par centrifugation et on récupère le culot cellulaire que l'on dissout dans de la soude 0,5 N.

On procède à la lecture de la densité optique sur un spectrophotomètre à 475 nm, ce qui permet d'évaluer la quantité de mélanine formée par comparaison à la densité optique d'une solution de mélanine de concentration connue.

On procède également au comptage des cellules, et on calcule le taux de mélanine formée par cellule, par rapport à une culture témoin, sans addition de produit à tester.

Chaque extrait de Coleus Esquirolii, de Coleus Scutellarioides, ou de Coleus Xanthanthus a été testé, à diverses concentrations en μg/ml, en utilisant comme témoin positif une culture recevant de la β-MSH (MELANINE STIMULATING HORMONE), à la concentration de $2.10^{-8}$ M.

L'activité A de stimulation de la mélanogénèse par les produits selon l'invention est calculée par la formule suivante :

$$A = \frac{q_p - q_o}{q_+ - q_o} \times 100$$

dans laquelle les grandeurs q représentent les quantités de mélanine formée

$q_p$ = culture recevant le produit à tester
$q_+$ = culture recevant la βMSH
$q_o$ = culture témoin ne recevant aucun produit.

L'activité de ces extraits, calculée selon la formule ci-dessus, figure aux tableaux I à III ci-après respectivement pour le Coleus Esquirolii, le Coleus Scutellarioides et le Coleus Xanthanthus.

TABLEAU I

| Extrait de Coleus Esquirolii (exemple 1) | | | | |
|---|---|---|---|---|
| Concentration produit μg/ml | nombres de cellules par boîte x $10^3$ | Mélanine μg pour $10^6$ cellules | Activité % | t |
| Témoin (aucun produit) | 169 ± 5 | 68 ± 4 | 0 | |
| β-MSH à $2.10^{-8}$M | 164 ± 5 | 136 ± 6 | 100 | |
| 2,5 | 159 ± 10 | 80 ± 4 | +18 | S |
| 5 | 164 ± 6 | 84 ± 3 | +23 | S |
| 10 | 156 ± 10 | 91 ± 3 | +34 | S |
| 25 | 155 ± 5 | 98 ± 8 | +44 | S |

TABLEAU II

| Extrait de Coleus Scutellarioides (exemple 2) | | | | |
|---|---|---|---|---|
| Concentration µg/ml | nombres de µg/ml de boîtes x $10^3$ | Mélanine µg pour $10^6$ cellules | Activité % | t |
| Témoin (aucun produit) | 161 ± 11 | 68 ± 6 | 0 | |
| β-MSH à $2.10^{-8}$M | 158 ± 7 | 138 ± 4 | 100 | |
| 2,5 | 166 ± 14 | 88 ± 5 | +28 | S |
| 5 | 161 ± 4 | 96 ± 3 | +40 | S |
| 10 | 159 ± 2 | 108 ± 2 | +57 | S |
| 25 | 183 ± 2 | 63 ± 3 | -7 | NS |

TABLEAU III

| Extrait de Coleus Xanthanthus (exemple 3) | | | | |
|---|---|---|---|---|
| Concentration produit µg/ml | Nombres de cellules par boîte x $10^3$ | Mélanine µg pour $10^6$ cellules | Activité % | t |
| Témoin (aucun produit) | 167 ± 7 | 45 ± 1 | 0 | |
| β-MSH à $2.10^{-8}$M | 162 ± 4 | 121 ± 1 | 100 | |
| 0,25 | 162 ± 13 | 51 ± 3 | +8 | NS |
| 1 | 149 ± 8 | 54 ± 3 | +12 | S |
| 2,5 | 162 ± 22 | 54 ± 4 | +12 | S |
| 10 | 165 ± 10 | 56 ± 4 | +14 | S |
| 25 | 176 ± 3 | 54 ± 3 | +12 | S |

Il résulte à partir des tableaux I à III ci-dessus que les extraits de Coleus selon l'invention stimulent la production de mélanine dans une proportion significative, ce qui représente un résultat tout à fait inattendu pour un homme de l'art.

On donnera ci-après divers exemples de formulation de composition cosmétique ou pharmaceutique, notamment dermatologique ayant une activité dans le traitement des désordres de la pigmentation cutanée.

EXEMPLE 7

Gel bronzant pour le visage

| | |
|---|---|
| Extrait de Coleus (poids sec) selon l'exemple 1 | 0,035 g |
| Ethanol | 40,- g |
| Eau distillée | 20,- g |
| Gel Carbopol® 940 à 1% | qsp 100 g |

EXEMPLE 8

Crème solaire bronzante

| | |
|---|---|
| Extrait de Coleus (poids sec) selon l'exemple 2 | 0,03g |
| Stéarate d'isocétyle | 8,- g |
| Huile d'arachide hydrogénée | 10,- g |
| Huile de lanoline | 3,5 g |
| Alcool cétylique | 5,- g |
| Alcool stéarylique | 2,5 g |
| Huile de vaseline légère | 10,- g |
| Monoester phosphorique de l'alcool cétylique OE neutralisé | 3,- g |
| Octylméthoxy cinnamate | 5,- g |

Cette phase est émulsionnée avec une phase aqueuse qsp 100 g contenant :

| | |
|---|---|
| Pantothénol | 0,1 g |
| Conservateurs | 0,2 g |

EXEMPLE 9

Lotion pour renforcer la protection solaire naturelle

| | |
|---|---|
| Alcool | 42,5 g |
| Propylèneglycol | 3,- g |
| Menthol | 0,05 g |
| Hydroxypropylméthylcellulose | 1,5 g |
| Extrait de Coleus (poids sec) selon l'exemple 2 | 0,03 g |
| Excipients aqueux parfumés | qsp 100 g |

Cette lotion est appliquée localement, de préférence deux fois par jour, quotidiennement pendant 3 à 8 jours précédant les expositions prolongées au soleil.
Les applications quotidiennes peuvent être poursuivies lors de la période d'exposition.

EXEMPLE 10

Lotion tonique capillaire contre les cheveux gris

| Extrait de Coleus selon l'exemple 3 | 0,02 g |
|---|---|
| IBMX | 0,1 g |
| Alcool | 30,- g |
| Eau | 69,- g |
| Excipients parfumés | qsp 100 g |

Cette lotion peut être appliquée sur les cheveux et le cuir chevelu deux fois par jour par cures de trois mois.

EXEMPLE 11

Gel dermatologique destiné à favoriser la pigmentation de la peau

| Extrait de Coleus selon l'exemple 3 | 0,03 g |
|---|---|
| Ethanol | 30 g |
| Eau distillée | 20 g |
| Gel de Carbopol® | qsp 100 g |

Ce gel est utilisé 1 à 2 fois par jour en application locale sur les zones de la peau à traiter.

EXEMPLE 12

Lotion capillaire contre les cheveux gris

| Extrait de Coleus, selon l'exemple 1 | 0,03 g |
|---|---|
| L-tyrosine éthyl ester, HCl | 1,0 g |
| Ethanol | 40,0 g |
| Parfum | 0,5 g |
| Solubilisant de parfum | 0,2 g |
| Eau qsp | 100 g |

Cette lotion, appliquée sur les cheveux et le cuir chevelu quotidiennement, permet de retarder l'apparition de cheveux gris.

EXEMPLE 13

Gel bronzant

| Extrait de Coleus, selon l'exemple 1 | 0,02 g |
|---|---|
| Filtre solaire (EUSOLEX 232 TS®) | 8,0 g |
| Ethanol | 40,0 g |
| Carbopol 940® | 1,0 g |
| Eau qsp | 100 g |

EXEMPLE 14

Lotion prévenant l'apparition des cheveux gris

| Suspension de liposome selon l'exemple 5 | 50 g |
|---|---|
| Carbopol 940® | 0,05 g |
| Tyrosinate de glucose | 0,05 g |
| Complexe d'oligo-éléments | 0,1 g |
| Théophylline | 0,01 g |
| Conservateur | 0,05 g |
| Eau distillée | qsp 100 ml |

On applique cette solution le soir sur le cuir chevelu au niveau des zones grisonnantes par cure de 4 mois.

**Revendications**

1. Composition cosmétique ou pharmaceutique, notamment dermatologique, caractérisée en ce qu'elle conprend à titre d'ingrédient actif un extrait de Coleus Esquirolii, de Coleus Scutellarioides, de Coleus Xanthanthus, ou un de leurs mélanges.

2. Composition cosmétique ou pharmaceutique, notamment dermatologique, destinée à favoriser la pigmentation de la peau ou des cheveux, caractérisée en ce qu'elle comprend à titre d'ingrédient actif un extrait de Coleus Esquirolii, de Coleus Scutellarioides, de Coleus Xanthanthus, ou un de leurs mélanges.

3. Composition selon la revendication 1 ou 2, caractérisée en ce que l'extrait de Coleus précité est un extrait organique de Coleus, de préférence obtenu par un procédé comprenant au moins une étape d'extraction avec un solvant choisi parmi le groupe constitué par l'acétate d'éthyle, le méthanol, l'éthanol ou le dichlorométhane.

4. Composition selon l'une des revendications 1 à 3, caractérisée en ce que la concentration en extrait de Coleus précité, exprimée en poids sec, est comprise entre 0,001 % et 2 % en poids, de préférence entre 0,01 % et 0,5 % en poids, par rapport au poids total de la composition.

5. Composition selon l'une des revendications précédentes, caractérisée en ce que la composition contient en outre une xanthine, en particulier l'IBMX (isobutylméthylxanthine) ou la théophylline, de préférence à une concentration pondérale comprise entre 0,01 et 2 %, et encore de préférence comprise entre 0,1 % et 0,5 %, par rapport au poids total de la composition.

**6.** Composition selon l'une des revendications précédentes, caractérisée en ce que la composition contient en outre de la tyrosine ou l'un de ses dérivés, de préférence à une concentration pondérale comprise entre 0,001 % et 10 %, par rapport au poids total de la composition.

**7.** Composition selon l'une des revendications précédentes, caractérisée en ce qu'elle contient en outre au moins une autre substance active, à une concentration efficace, choisie parmi les vitamines, en particulier les vitamines B, la quinine ou ses dérivés, les rubéfiants, tels que le nicotinate de méthyle, un surnageant de culture de fibroblastes de papilles, des hydrolysats de kératine, des oligo-éléments tels que zinc, sélénium, cuivre, des inhibiteurs de 5 $\alpha$-réductase, tels que progestérone, cyprotérone acétate, Minoxidil, acide azélaïque et des dérivés, un 4-méthyl-4-azastéroïde, en particulier la 17-$\beta$-N,N-diéthylcarbamoyl-4-méthyl-4-aza-5-$\alpha$-androstan-3-one, ou encore un extrait de Serenoa repens.

**8.** Composition selon l'une des revendications précédentes, caractérisée en ce qu'on formule la composition sous une forme permettant une application topique, en particulier sous forme de crème, de gel ou de lotion destiné à l'application sur la peau ou sur les cheveux.

**9.** Procédé de traitement cosmétique de la peau ou des cheveux pour favoriser la pigmentation, caractérisé en ce qu'il comprend l'application en une quantité efficace pour réaliser une pigmentation, d'au moins un extrait de Coleus Esquirolii, de Coleus Scutellarioides, de Coleus Xanthanthus, ou d'un de leurs mélanges, dans un excipient, véhicule ou support cosmétiquement acceptable.

**10.** Procédé selon la revendication 9, caractérisé en ce que l'extrait de Coleus précité est un extrait organique de Coleus, de préférence obtenu par un procédé comprenant au moins une étape d'extraction avec l'acétate d'éthyle.

**11.** Procédé selon l'une des revendications 9 ou 10, caractérisé en ce que l'on incorpore en outre, dans l'excipient, véhicule ou support, une quantité efficace d'au moins un composé choisi parmi les xanthines, en particulier l'IBMX (isobutylméthylxanthine) ou la théophylline, la tyrosine ou l'un de ses dérivés, les vitamines, en particulier les vitamines B, la quinine ou ses dérivés, les rubéfiants, tels que le nicotinate de méthyle, un surnageant de culture de fibroblastes de papilles, des hydrolysats de kératine, des oligo-éléments tels que zinc, sélénium, cuivre, des inhibiteurs de 5 $\alpha$-réductase, tels que progestérone, cyprotérone acétate, Minoxidil, acide azélaïque et ses dérivés, un 4-méthyl-4-azastéroïde, en particulier la 17-$\beta$-N,N-diéthylcarbamoyl- 4-méthyl-4-aza-5-$\alpha$-androstan-3-one, ou encore un extrait de Serenoa repens.

**12.** Utilisation d'un extrait de Coleus Esquirolii, de Coleus Scutellarioides, de Coleus Xanthanthus ou d'un de leurs mélanges, pour la préparation d'une composition cosmétique ou pharmaceutique, notamment dermatologique, destinée à favoriser la pigmentation de la peau ou des cheveux et/ou au traitement des désordres de la pigmentation de la peau et des cheveux.

**13.** Utilisation selon la revendication 12, caractérisée en ce que l'extrait de Coleus précité est un extrait organique obtenu par un procédé comprenant au moins une étape d'extraction avec l'acétate d'éthyle.

**14.** Utilisation selon la revendications 12 ou 13, caractérisée en ce que la composition précitée contient en outre une quantité efficace d'au moins une autre substance active, choisie parmi les xanthines, en particulier l'IBMX (isobutylméthylxanthine) ou la théophylline, la tyrosine ou l'un de ses dérivés, les vitamines, en particulier les vitamines B, la quinine ou ses dérivés, les rubéfiants, tels que le nicotinate de méthyle, un surnageant de culture de fibroblastes de papilles, des hydrolysats de kératine, des oligo-éléments tels que zinc, sélénium, cuivre, des inhibiteurs de 5 $\alpha$-réductase, tels que progestérone, cyprotérone acétate, Minoxidil, acide azélaïque et ses dérivés, un 4-méthyl-4-azastéroïde, en particulier la 17-$\beta$-N,N-diéthylcarbomoyl-4-méthyl-4-aza-5-$\alpha$-androstan-3-one, ou encore un extrait de Serenoa repens.

**Claims**

**1.** Cosmetic or pharmaceutical composition, especially dermatological composition, characterized in that it comprises, as active ingredient, an extract of Coleus Esquirolii, Coleus Scutellarioides or Coleus Xanthanthus, or a mixture thereof.

**2.** Cosmetic or pharmaceutical composition, especially dermatological composition, intended for promoting the pigmentation of the skin or hair, characterized in that it comprises, as active ingredient, an extract of Coleus Esquirolii, Coleus Scutellarioides or Coleus Xanthanthus, or a mixture thereof.

3. Composition according to claim 1 or 2, characterized in that the above-mentioned extract of Coleus is an organic extract of Coleus, preferably obtained by a process comprising at least one extraction step with a solvent selected from the group consisting of ethyl acetate, methanol, ethanol or dichloromethane.

4. Composition according to one of claims 1 to 3, characterized in that the concentration of the above-mentioned extract of Coleus, expressed by dry weight, is between 0.001% and 2% by weight, preferably between 0.01% and 0.5% by weight, with respect to the total weight of the composition.

5. Composition according to one of the preceding claims, characterized in that the composition also contains a xanthine, in particular IBMX (isobutylmethylxanthine) or theophylline, preferably at a concentration by weight of between 0.01% and 2% and particularly preferably of between 0.1% and 0.5%, with respect to the total weight of the composition.

6. Composition according to one of the preceding claims, characterized in that the composition also contains tyrosine or a derivative thereof, preferably at a concentration by weight of between 0.001% and 10%, with respect to the total weight of the composition.

7. Composition according to one of the preceding claims, characterized in that it further contains an effective concentration of at least one other active substance selected from vitamins, in particular the B vitamins, quinine or derivatives thereof, rubefacients such as methyl nicotinate, a papilla fibroblast culture supernatant, keratin hydrolyzates, trace elements such as zinc, selenium and copper, 5-$\alpha$-reductase inhibitors such as progesterone, cyproterone acetate and minoxidil, azelaic acid and derivatives thereof, a 4-methyl-4-azasteroid, in particular 17-$\beta$-N,N-diethylcarbomoyl-4-methyl-4-aza-5-$\alpha$-androstan-3-one, or else an extract of Serenoa repens.

8. Composition according to one of the preceding claims, characterized in that the composition is presented in a form permitting topical application, in particular in the form of a cream, a gel or a lotion intended for application to the skin or hair.

9. Process for the cosmetic treatment of the skin or hair intended for promoting the pigmentation, characterized in that it comprises the application, in an effective amount to carry out the pigmentation, of at least one extract of Coleus Esquirolii, Coleus Scutellarioides or Coleus Xanthanthus, or a mixture thereof, into a cosmetically or pharmaceutically acceptable excipient, vehicle or carrier.

10. Process according to claim 9, characterized in that the above-mentioned extract of Coleus is an organic extract of Coleus, preferably obtained by a process comprising at least one extraction step with ethyl acetate.

11. Process according to one of claims 9 or 10, characterized in that an effective amount of at least one compound selected from xanthines, in particular IBMX (isobutylmethylxanthine) or theophylline, tyrosine or a derivative thereof, vitamins, in particular the B vitamins, quinine or derivatives thereof, rubefacients such as methyl nicotinate, a papilla fibroblast culture supernatant, keratin hydrolyzates, trace elements such as zinc, selenium and copper, 5-$\alpha$-reductase inhibitors such as progesterone, cyproterone acetate and minoxidil, azelaic acid and derivatives thereof, a 4-methyl-4-azasteroid, in particular 17-$\beta$-N,N-diethylcarbamoyl-4-methyl-4-aza-5-$\alpha$-androstan-3-one, or else an extract of Serenoa repens, is also incorporated in the excipient, vehicle or carrier.

12. Use of an extract of Coleus Esquirolii, Coleus Scutellarioides or Coleus Xanthanthus, or a mixture thereof, for the preparation of a cosmetic or pharmaceutical composition, especially dermatological composition, intended for promoting the pigmentation of the skin or hair and/or for treating pigmentation disorders of the skin and hair.

13. Use according to claim 12, characterized in that the above-mentioned extract of Coleus is an organic extract obtained by a process comprising at least one extraction step with ethyl acetate.

14. Use according to claim 12 or 13, characterized in that the above-mentioned composition also contains an effective amount of at least one other active substance selected from xanthines, in particular IBMX (isobutylmethylxanthine) or theophylline, tyrosine or a derivative thereof, vitamins, in particular the B vitamins, quinine or derivatives thereof, rubefacients such as methyl nicotinate, a papilla fibroblast culture supernatant, keratin hydrolyzates, trace elements such as zinc, selenium and copper, 5-$\alpha$-reductase inhibitors such as progesterone, cyproterone acetate and minoxidil, azelaic acid and derivatives thereof, a 4-methyl-4-azasteroid, in particular 17-$\beta$-N,N-diethylcarbamoyl-4-methyl-4-aza-5-$\alpha$-androstan-3-one, or else an extract of Serenoa repens.

**Patentansprüche**

1. Kosmetische oder pharmazeutische, insbesondere dermatologische, Zusammensetzung, dadurch gekennzeichnet, daß sie als aktiven Bestandteil einen Extrakt von Coleus esquirolii, Coleus scutellarioides, Coleus xanthantus oder einer ihrer Mischungen enthält.

2. Kosmetische oder pharmazeutische, insbesondere dermatologische, Zusammensetzung zur Förderung der Pigmentierung der Haut oder der Haare, dadurch gekennzeichnet, daß sie als aktiven Bestandteil einen Extrakt von Coleus esquirolii, Coleus scutellarioides, Coleus xanthantus oder einer ihrer Mischungen enthält.

3. Zusammensetzung nach Anspruch 1 oder 2, dadurch gekennzeichnet, daß der vorgenannte Coleus-Extrakt ein organischer Coleus-Extrakt ist, der vorzugsweise durch ein Verfahren erhalten wird, das zumindest einen Extraktionsschritt mit einem Lösungsmittel, ausgewählt aus der Gruppe bestehend aus Ethylacetat, Methanol, Ethanol oder Dichlormethan, umfaßt.

4. Zusammensetzung nach einem der Ansprüche 1 bis 3, dadurch gekennzeichnet, daß die Konzentration des vorgenannten Coleus-Extrakts, ausgedrückt als Trockenmasse, zwischen 0,001 Masse-% und 2 Masse-%, vorzugsweise zwischen 0,01 Masse-% und 0,5 Masse-%, bezogen auf die Gesamtmasse der Zusammensetzung, beträgt.

5. Zusammensetzung nach einem der vorhergehenden Ansprüche, dadurch gekennzeichnet, daß die Zusammensetzung ferner ein Xanthin, insbesondere IBMX (Isobutylmethylxanthin) oder Theophyllin, vorzugsweise in einer Massekonzentration zwischen 0,01 und 2 %, und auch vorzugsweise zwischen 0,1 % und 0,5 %, bezogen auf die Gesamtmasse der Zusammensetzung, enthält.

6. Zusammensetzung nach einem der vorhergehenden Ansprüche, dadurch gekennzeichnet, daß die Zusammensetzung ferner Tyrosin oder eines seiner Derivate, vorzugsweise in einer Massekonzentration zwischen 0,001 % und 10 %, bezogen auf die Gesamtmasse der Zusammensetzung, enthält.

7. Zusammensetzung nach einem der vorhergehenden Ansprüche, dadurch gekennzeichnet, daß sie ferner zumindest eine andere aktive Substanz, in einer wirksamen Konzentration, ausgewählt aus Vitaminen, insbesondere Vitamin B, Chinin oder seinen Derivaten, Rubefacientien, wie Methylnicotinat, einem Kulturüberstand von Papillenfibroblasten, Keratinhydrolysaten, Oligoelementen, wie Zink, Selen, Kupfer, 5-$\alpha$-Reduktase-Inhibitoren, wie Progesteron, Cyproteronacetat, Minoxidil, Azelainsäure und ihren Derivaten, einem 4-Methyl-4-azasteroid, insbesondere 17$\beta$-N,N-Di-ethylcarbamoyl-4-methyl-4-aza-5-$\alpha$-androstan-3-on, oder auch einen Extrakt von Serenoa repens enthält.

8. Zusammensetzung nach einem der vorhergehenden Ansprüche, dadurch gekennzeichnet, daß die Zusammensetzung in einer Form, die eine topische Anwendung ermöglicht, insbesondere in Form einer Creme, eines Gels oder einer Lotion, zum Aufbringen auf die Haut oder auf die Haare formuliert ist.

9. Kosmetisches Verfahren zur Behandlung der Haut oder der Haare zur Förderung der Pigmentierung, dadurch gekennzeichnet, daß es das Aufbringen, in einer wirksamen Menge zum Bewirken einer Pigmentierung, zumindest eines Extrakts von Coleus esquirolii, Coleus scutellarioides, Coleus xanthantus oder einer ihrer Mischungen in einem kosmetisch annehmbaren Exzipienten, Vehikel oder Träger umfaßt.

10. Verfahren nach Anspruch 9, dadurch gekennzeichnet, daß der vorgenannte Coleus-Extrakt ein organischer Coleus-Extrakt ist, der vorzugsweise durch ein Verfahren erhalten wird, das zumindest einen Extraktionsschritt mit Ethylacetat umfaßt.

11. Verfahren nach einem der Ansprüche 9 oder 10, dadurch gekennzeichnet, daß ferner, in einem Exzipienten, Vehikel oder Träger, eine wirksame Menge zumindest einer Verbindung, ausgewählt aus Xanthinen, insbesondere IBMX (Isobutylmethylxanthin) oder Theophyllin, Tyrosin oder einem seiner Derivate, Vitaminen, insbesondere Vitamin B, Chinin oder seinen Derivaten, Rubefacientien, wie Methylnicotinat, einem Kulturüberstand von Papillenfibroblasten, Keratinhydrolysaten, Oligoelementen, wie Zink, Selen, Kupfer, 5-$\alpha$-Reduktase-Inhibitoren, wie Progesteron, Cyproteronacetat, Minoxidil, Azelainsäure und ihren Derivaten, einem 4-Methyl-4-azasteroid, insbesondere 17$\beta$-N,N-Diethylcarbamoyl-4-methyl-4-aza-5-$\alpha$-androstan-3-on, oder auch eines Extrakts von Serenoa repens eingeschlossen wird.

12. Verwendung eines Extrakts von Coleus esquirolii, Coleus scutellarioides, Coleus xanthantus oder einer ihrer Mischungen bei der Herstellung einer kosmetischen oder pharmazeutischen, insbesondere dermatologischen, Zusammensetzung zur Förderung der Pigmentierung der Haut oder der Haare und/oder zur Behandlung von Pigmentstörungen der Haut und der Haare.

13. Verwendung nach Anspruch 12, dadurch gekennzeichnet, daß der vorgenannte Coleus-Extrakt ein organischer Extrakt ist, der vorzugsweise durch ein Verfahren erhalten wird, das zumindest einen Extraktionsschritt mit Ethylacetat umfaßt.

14. Verwendung nach Anspruch 12 oder 13. dadurch gekennzeichnet, daß die vorgenannte Zusammensetzung ferner eine wirksame Menge zumindest einer anderen aktiven Substanz, ausgewählt aus Xanthinen, insbesondere IBMX (Isobutylmethylxanthin) oder Theophyllin, Tyrosin oder einem seiner Derivate, Vitaminen, insbesondere Vitamin B, Chinin oder seinen Derivaten, Rubefacientien, wie Methylnicotinat, einem Kulturüberstand von Papillenfibroblasten, Keratinhydrolysaten, Oligoelementen, wie Zink, Selen, Kupfer, 5-$\alpha$-Reduktase-Inhibitoren, wie Progesteron, Cyproteronacetat, Minoxidil, Azelainsäure und ihren Derivaten, einem 4-Methyl-4-azasteroid, insbesondere 17$\beta$-N,N-Diethylcarbamoyl-4-methyl-4-aza-5-$\alpha$-androstan-3-on, oder auch eines Extrakts von Serenoa repens enthält.